# EUROPEAN PATENT APPLICATION

(11) **EP 3 085 356 A1**
(43) Date of publication of application: **26.10.2016**
(21) Application number: 16468001.9
(22) Date of filing: 22.04.2016
(51) Int. Cl.: A61K 8/98, A61K 35/644, A23L 21/25, A23L 33/105

(54) **HONEY WITH PLANT EXTRACTS AND PRODUCTION METHOD THEREOF**

(30) Priority: 24.04.2015 SI 201500107
(71) Applicant: Borut, Gosar, 1217 Vodice (SI)
(72) Inventor: Borut, Gosar, 1217 Vodice (SI)

(57) **Abstract**

Honey with the plant extracts, and the production process. Such honey is prepared in the multiphase process. In the final stage redundant solvent is slowly eliminated at low temperature in vacuum and continuous stirring while the honey-insoluble bioactive compounds are gradually bind to honey. Honey with the plant extracts is produced in such manner that the bioactive compounds are allocated in honey so that the honey does not lose its basic physicochemical and microbiological properties. The honey obtained does not require the additives, has appropriate density, moisture content, is microbiologically stable, but also contains a high content of plant bioactive compounds, which are dissolved in honey or uniformly distributed.

## Description

Honey with plant extracts is prepared in such a manner that in the honey-insoluble bioactive compounds from the plants are relocated in the honey so that honey does not lose its physical and microbiological characteristics. This honey does not require additives, has the appropriate density and moisture content, is microbiologically stable, while also contains a high content of plant bioactive compounds, which are and remain in the honey dissolved or evenly dispersed.

### Technical field to which the invention relates;

Subject of the invention is honey with plant extracts, which are not soluble in water or honey and its production process. This honey contains a high concentration of bioactive compounds from plants that are used to help relieve cough, to relieve pain in the throat, to eliminate and treat sore throat, inflamed mucous, inflamed skin, as an antibacterial protection, to facilitate expectoration, etc.

According to the International Patent Classification invention falls into the category of A23L.

### Outlook of the technical problem solved by this invention for which is claimed patent protection;

The technical problem is to make the product on the basis of honey with a high content of plant bioactive compounds (oil, essential oils, resins, mucus, heteroglycan polysaccharides, organic acids, amino acids, isoprenoides, aromatic compounds, flavonoids, bitter substances, alkaloids, tannins, vitamins, inorganic substances, etc.), which are not soluble or are poorly soluble in the honey and water. The product on the basis of honey must have the bioactive compounds from the plant evenly dispersed or dissolved, and should have similar physicochemical and microbiological parameters as honey. It must contain less than 20% of water, microbiological must remain stable and must not spoil.

### State of the art, known existing solutions and their weaknesses

Liquid extracts from medicinal plants have long been used to boost immunity, to relieve cough and sore throat, to treat inflammation, wounds, mental disorders, etc. Liquid extracts can be used to prepare various syrups by the different methods. The disadvantage of syrups is that they contain a lot of water and are therefore subjected to deterioration or contain possibly harmful substances (ethanol and other solvents, sugar, antioxidants, inhibitors of growth of micro-organisms) that, during storage and after opening do not spoil.

Well-known recipe is the preparation of sugar-based spruce tips. In the glass we put several layers of tips and sugar and put the content for a few weeks on the sun. Sugar due to the high hygroscopicity and osmosis eliminates water from the tips together with their beneficial compounds (essential oils, resins, antioxidants, vitamins) from the tips. Therefore, the content modified from the solid to a liquid state or syrup, respectively.

Beekeepers can prepare syrups from medicinal herbs on the basis of honey. It is prepared with the procedure that fresh plants are soaked in honey with lesser content of water. The content is mixed every few days and after a few weeks mixture is filtered. The advantage of honey-based syrups, compared to those based on sugar is that they are more pleasant taste, and contain more antioxidants and vitamins, because these syrups are not subjected to heat treatment. Since the bioactive compounds from plants usually do not dissolve or dissolve poorly in honey and water, these syrups contain insufficient bioactive compounds and an excessive amount of water, which may spoil the syrup. Beekeepers can limit water problem by drying buds before they are added to honey, but this reduces the absorption of bioactive compounds from the plant material in to the honey.

With the development of various vacuum techniques and instruments we can reduce the water content in honey so that higher water content does not affect on the honey quality. Among the most suitable methods to remove water from honey is low-temperature vacuum distillation process. Here with vacuum distiller (Rota vapor) at temperature of 40 °C and vacuum, excess water from the honey is removed, without negative effects on the quality. Such honey is otherwise microbiologically stable but does not contain a lot of oil, essential oils, resins and other plant bioactive compounds that are not soluble in water or honey.

### Description of new invention

The solution for that technical problem is multiphase process of preparing such a honey with bioactive compounds from plants (echinacea, St. John's wort, lemon balm, mint, thyme, sage, plantain, tips or apices of plants from genus spruce (*Picea sp.),* fir (*Abies sp),* pine *(Pinus sp)),* which are used in food or in traditional medicine for the treatment and elimination of most diverse diseases and health problems.

Primary the plants are collected, if necessary grinded and fresh or dried soaked in solvent, which is most suitable for extraction of the targeted active compounds. Most natural and accessible solvent for the extraction of the majority of bioactive compounds from plants is ethanol and water. There are also other solvents, such as propanol, isopropanol, ethyl acetate, acetone, propane, hexane, etc. Each of them is suitable for dissolving specific bioactive compound (oil, essential oils, resins, mucus, heteroglycanic polysaccharides, organic and amino acids, isoprenoids, aromatic compounds, flavonoids, bitter substances, alkaloids, tannins, vitamins, inorganic materials, etc.).

Plants are soaked in the solvent for a certain time. Then, by pressing and filtering they are separated from the liquid (extract) which contain dissolved bioactive compounds. In the extract obtained, honey is added in varying amounts, primarily from 10% to 90% by the weight of the extract. The extract and honey is mixed until complete solubility. Solubility is achieved if honey and extract are soluble in each other.

The resulting mixture is now comparable to syrup. If we want to remove excess of the solvent (i.e., ethanol or water) from the syrup, this should be done by distillation. As honey and the majority of bioactive compounds should not be heated above 60 °C, the distillation process should be done at a temperature up to 60 °C. Preferably the temperature is better not to exceed 40 °C. The method that enables this is vacuum distillation, where in the vacuum access solvents (eg, ethanol and water) can be evaporated at a temperature of 40 °C. The most suitable instrument for evaporating excess solvents is a rotating vacuum distiller.

In this process in almost absolute vacuum and constant stirring the solvent (such as ethanol and water or both) is removed from the solution. When a solvent (for example, ethanol in which are dissolved plant substance) is no longer present in the solution, the plant bioactive compounds (for example, essential oils, oils, resins, slime, ...) according to this method effectively bind to the honey, due to the continuous mixing and slow solvent removing. When the appropriate amount of solvent is evaporated, honey contain dissolved plant bioactive compounds and regains its typical physic and chemical properties. It becomes homogeneous, adequate density and with water content below 20%. As such is microbiologically stable, and therefore do not require further treatment or additives against possible spoilage.

## Claims

1. Honey with plant extracts, **characterized in that** it contains honey and a plant extract obtained from buds of species of genus spruce, *Picea sp.,* fir *Abies sp.* or pine *Pinus sp.* or their mixtures, or a plant extract obtained from plants echinacea, St. John's wort, balm, mint, thyme, sage or plantain, wherein the content of honey in the starting mixture is from 10 to 90 wt. % based on the weight of plant extract.

2. Method for the production of honey with plant extracts according to claim 1, **characterized in that** it includes the following phases:
- Extraction of whole or grinded, dried or fresh plant in a solvent,
- Separation of plant parts from liquid plant extract by pressing and filtration,
- Addition of honey in a plant extract in an amount varying from 10 to 90 % by weight with continuous stirring,
- Removing the excess solvent from the mixture of honey and plant extract by distillation under vacuum at a temperature below 60 °C with constant stirring, until the water content in honey is below 20 wt. %, wherein in the honey-insoluble plant bioactive compounds that are present in the extract are due to the continuous mixing and slow solvent removal bind to honey and form a homogenous mixture (solution) of honey and dissolved plant active ingredients, wherein honey retains its physical, chemical and microbiological properties.

3. Method according to claim 2, **characterized in that** the solvent is ethanol, propanol, isopropanol, ethyl acetate, acetone, propane or hexane.

4. Method according to claim 2, **characterized in that** the solvent is mixture of ethanol and water.

5. Method according to claims 2 to 4, **characterized in that** removal of redundant solvent takes place in a rotary vacuum distiller at a temperature below 60 °C, preferably at temperature of 40 °C.

6. Method according to claims 2 to 5, **characterized in that** the plant extracts are obtained from the buds of species belonging to genus spruce, *Picea sp.,* fir *Abies sp.* or pine *Pins sp.* or their mixtures.

7. Method according to claims 2 to 5, **characterized in that** the plant extracts are obtained from plants echinacea; St. John's wort, balm, mint, thyme, sage or plantain.

8. Honey with plant extracts are obtained by the method according to claims 2 to 7.
